# EUROPEAN PATENT APPLICATION

(11) **EP 2 638 909 A1**
(43) Date of publication of application: **18.09.2013**
(21) Application number: 12159737.1
(22) Date of filing: 15.03.2012
(51) Int. Cl.: A61K 31/542

(54) **Process for preparing sterile Brinzolamide**

(71) Applicant: Azad Pharmaceutical Ingredients AG, 8200 Schaffhausen (CH)
(72) Inventor: Porstmann, Frank, 8234 Stetten (CH); Ondracek, Jan, 8207 Schaffhausen (CH); Khov-Tran, Van Van, 8808 Pfäffikon SZ (CH); Grams, Gregory, 4430 Ans (BE)
(74) Representative: Weiss, Wolfgang

(57) **Abstract**

Process of preparing sterile brinzolamide using ethylene-oxide gas sterilisation, sterile brinzolamide obtainable by said process and pharmaceutical compositions comprising said sterile brinzolamide.

## Description

The invention refers to a of preparing sterile active pharmaceutical ingredient (API), sterile API obtainable by process and pharmaceutical compositions containing said sterile API.

Brinzolamide [(4R)-4-(ethylamino)-3,4-dihydro-2-(3-methoxypropyl)-2H-thieno[3,2-e]-1,2-thiazine-6-sulfonamide-1,1 -dioxide] is a carbonic anhydrase inhibitor useful for the treatment of ocular hypertension and glaucoma. The product and its manufacture is described e.g. in US 5 240 923, US 5 378 703 and US 5 461 and is commercially available. Regarding the manufacture of brinzolamide, it is particularly to the disclosure of WO 2010/103115, more particularly to claims 1, 7-10 and 12-14 of WO 2010/103115, which are herein incorporated by reference.

It is known that brinzolamide usually if subjected to conventional sterilization processes. US 6,071,904 and EP 0941 094 describe that radiation in an unacceptable degradation of the brinzolamide product levels. It also that sterilisation of brinzolamide product by means of ethylene oxide sterilisation processes introduces unacceptable degradation products and residues. From the above it was conduced aseptic ball milling in combination with heat sterilization to be the only viable method to manufacture sterile ophthalmic brinzolamide suspensions.

EP 2 394 637 discloses sterilization of brinzolamide using pure ethylene-under reduced pressure. The sterilisation process over 33 hours. The is said to in low amounts of (S)-4-(ethylamino)-3,4-dihydro-2-(3-methoxypropyl)-2H-thieno[3,2e]-1,2-thiazine-6-sulfonamide-1,1-dioxide. EP 2 394 637 does not teach anything about formation of other structurally related impurities. It focuses on the absence of formation of the Brinzolamide enantiomer.

It has now been found that by variation of the conditions of the sterilisation process, the reaction times could be significantly reduced. Moreover, the such modified process resulted not only in reduced amounts of the brinzolamide enantiomer (S)-4-(ethylamino)-3,4-dihydro-2-(3-methoxypropyl)-2H-thieno[3,2e]-1,2-thiazine-6-sulfonamide-1,1-dioxide, but also in reduced amounts of degradation products formed during the sterilization process.

Thus, in one aspect the present invention refers to a process for sterilising API comprising the steps of (i) providing an API product and (ii) contacting said API product with ethylene-oxide, under conditions that at most 0.5 %, preferably at most 0.3 %, more preferably at most 0.15 % by weight of degradation products are formed during step (ii), based on the total amount of sterilized product.

The active pharmaceutical ingredient (API) may be brinzolamide, nepafenac, or levocabastine hydrochloride, most preferably brinzolamide. In a preferred embodiment the API is present as a powder. The powder has usually a particle size distribution in the range of 0.2 µm ≤ d_{90%} ≤ 5.0 µm, preferably 0.2 µm ≤ d_{90%} ≤ 4.0 µm, and more preferably 1.0 µm ≤ d_{90%} ≤ 3.5 µm.

When carrying out the sterilisation via gas vapour ethylene-oxide sterilisation under certain conditions, it has been surprisingly found that the API, such as brinzolamide, withstands the ethylene-oxide sterilization conditions without significant degradation or rearrangement tendency of the product resulting in a high purity sterile API, e.g. brinzolamide.

"*Sterilisation*", "*sterilising*" or "*sterile*" as herein used means that the probability of finding one (1) augmentable microorganism in a sample unit is (reduced to) at most 10⁻⁶.

"*Degradation produce*" as herein used means any compound other than API, such as brinzolamide, which is formed during the sterilisation step (ii). It is known that the enantiomer of brinzolamide (S)-4-(ethylamino)-3,4-dihydro-2-(3-methoxy-propyl)-2H-thieno[3,2e]-1,2-thiazine-6-sulfonamide-1,1-dioxide may be generated during the sterilization step due to epimerization or racemization reactions in minor amounts. However, according to the present invention the brinzolamide enantiomer (S)-4-(ethylamino)-3,4-dihydro-2-(3-methoxypropyl)-2H-thieno[3,2e]-1,2-thiazine-6-sulfonamide-1,1 1-dioxide is not regarded as a "degradation product".

In a preferred embodiment ethylene-oxide sterilization is carried out at temperatures of between 30-80°C, preferably 40-70°C, preferably 45-60°C, more preferably 50-60°C.

The ethylene oxide concentrations may be adjusted to 100-800 mg/l, preferably 200-600 mg/l, preferably 350-600 mg/l and most preferably 300-450 mg/l.

Contacting times in step (ii) are preferably about 15-200 min., preferably 20-180 min., preferably 20-100 min., and more preferably 40-80 min.

The relative humidity when carrying out step (ii) is preferably in the range of about 30-70 %, more preferably 40-70%, and most preferably about 40-60%.

In one embodiment, the sterilisation process may be combined with other sterilisation methods such as heat or gas (different from ethylene-oxide gas) sterilisation and/or sterilisation by ionizing radiation. Heat sterilisation, preferably includes moist and dry heat sterilisation. Ionizing radiation, preferably includes gamma radiation and electron-beam treatment. Gas sterilisation preferably includes sterilisation by other gases than ethylene-oxide, such as e.g. hydrogen peroxide.

Another aspect of the invention is a sterile API, such as brinzolamide, obtainable by the above described process.

In a preferred embodiment the sterilized product contains equal to or less than 0.5 % by weight, preferably equal to or less than 0.3 % by weight, more preferably equal to or less than 0.15 % by weight of degradation products directly after ethylene-oxide sterilization.

Moreover the sterilized product is essentially free of ethylene oxide. For example, the sterilized product comprises ethylene oxide in an amount of at most 10 ppm, preferably at most 3 ppm, and more preferably at most 1 ppm directly after sterilization. However, traces of ethylene oxide cannot always be avoided when sterilizing the obtained product via gas-vapour ethylene-oxide sterilization so that the sterilized products may comprise 0.01 ppb, or 0.1 ppb, or even 1 ppb of ethylene-oxide in the obtained product.

In another aspect, the present invention refers to a pharmaceutical composition, particularly for ophthalmic use, comprising the sterile API, such as brinzolamide, obtainable by the above described sterilisation process.

In a preferred embodiment pharmaceutical compositions, particularly for ophthalmic use are provided, comprising the sterile API, such as brinzolamide, obtainable by the above described sterilisation process, at least one preservative, at least one isotonicity agent, at least one viscosity enhancing agent, at least one sequestering agent and water.

The pharmaceutical compositions comprise API, such as brinzolamide, preferably in amounts of 5-20 mg/ml, preferably 5-15 mg/ml, more preferably 8-12 mg/ml.

In one preferred embodiment, the preservative, if present, is selected from, but not limited to, the group consisting of benzalkonium chloride, benzoic acid, benzyl alcohol, butylparaben, propylparaben, methylparaben, chlorobutanol, phenoxyethanol. Preferably, the preservative is benzalkonium chloride. Typically, the total amount of the preservative(s) in the pharmaceutical composition may vary in the range of 0.01-1.0 mg/ml, more preferably 0.05-0.5 mg/ml and even more preferably 0.05-0.2 mg/ml.

The isotonicity agent according to the present invention is preferably selected from, but not limited to, the group consisting of dextrose, glycerol, mannitol, potassium chloride and sodium chloride. Preferably, the isotonicity agent is mannitol and/or sodium chloride, more preferably mannitol and sodium chloride. Typically, the isotonicity agents may be present in total amounts of 15-80 mg/ml, more preferably 20-50 mg/ml, even more preferably 25-50 mg/ml.

According to the present invention, the viscosity enhancing agent is preferably selected from, but not limited to, the group consisting of carbomer, poloxamer, polyvinyl alcohol, povidone, polyethylene oxide, carboxymethylcellulose calcium, carboxymethylcellulose sodium, hydroxyethylcellulose, hydroxypropylcellulose, hydroxymethylcellulose, hydroxypropylmethylcellulose, and methylcellulose. In one particularly preferred embodiment, the viscosity enhancing agent is carbomer. Typically, the viscosity enhancing agents may be present in total amounts of 0.5-10 mg/ml, preferably 1-8 mg/ml, more preferably 2-6 mg/ml.

The sequestering agent according to the invention is preferably a chelator, which forms a chelate complex with cations, such as EDTA (ethylenediaminetetraacetic acid) and salts thereof. The sequestering agent may be present in amounts of 0.01-2 mg/ml, preferably 0.05-1.5 mg/ml, more preferably 0.07-0.5 mg/ml.

The water used in the pharmaceutical compositions of the invention fulfils the requirements for ophthalmic administration.

Optionally, further carriers and/or diluents may be present in the pharmaceutical composition.

Preferred carriers are e.g. buffering agents or pH-adjusting agents. Buffering agents include preferably phosphate buffers, borate buffers and citrate buffers. pH-adjusting agents include preferably aqueous sodium hydroxide (NaOH) and hydrochloric acid (HCl). In a preferred embodiment, the pharmaceutical composition of the invention is adjusted by pH-adjusting agents to a pH between 5.0-8.0 preferably to a pH of 7-8. In most preferred embodiments, the pH of the composition is substantially 7.3-7.7 (physiological).

Due to the low solubility of API, such as brinzolamide, in water the pharmaceutical composition of the invention is preferably a suspension, that is, API particles, such as brinzolamide particles, are homogeneously distributed in the liquid medium. The particle size distribution d_{90%} is preferably less than 10 µm, since higher values usually cause irritations. Thus, in a preferred embodiment, the particle size distribution is 0.2 µm ≤ d_{90%} ≤ 5.0 µm, more preferably 0.2 µm ≤ d_{90%} ≤ 4.0 µm, and even more preferably 1.0 µm ≤ d_{90%} ≤ 3.5 µm.

In one preferred embodiment, the pharmaceutical composition according to the invention is free of surfactant, particularly free of non-ionic, cationic or anionic surfactants, more preferably free of non-ionic surfactants. Surfactants which are preferably absent in the compositions of the invention are e.g. Tyloxapol, TritonX-100, sodium laurylsulfate, docusate sodium, polyoxyalkylethers, polyoxyalkyl phenyl ethers, polyoxyhydrogenated castor oil, polyoxysorbitan esters and/or sorbitane esters. In a most preferred embodiment the pharmaceutical composition according to the present invention is free of Tyloxapol.

It has been surprisingly found that despite the absence of a surfactant, the particle size of API, such as brinzolamide, in the pharmaceutical compositions of the invention is not increased, suggesting that the surfactant is not required to homogeneously suspend the solid API particles, such as brinzolamide particles, within the liquid matrix of the pharmaceutical composition. Moreover, the suspension stability and the resuspendability of the pharmaceutical compositions having no surfactant are comparable to those of formulations having an additional surfactant, such as Tyloxapol. Accordingly, use of surfactants can be avoided without influencing the stability properties of the pharmaceutical composition. It follows that the pharmaceutical compositions of the present invention are simple to manufacture and minimize the presence of potentially harmful surfactant components.

The sterilisation process allows a straight forward formulation procedure by means of aseptic mixing of sterile API products, such as brinzolamide products, with the further components of the pharmaceutical composition.

Thus, in another aspect the present invention provides a method for preparing a sterile API composition comprising the steps of
(i) preparing a carbomer dispersion in water,
(ii) preparing a solution comprising at least one isotonicity agent, at least one chelator and optionally at least one preservative in water,
(iii) adding the solution of step (ii) into the dispersion of step (i),
(iv) adjusting the pH,
(v) sterilising the mixture obtained in step (iv),
(vi) adding sterile API, such as brinzolamide, as micronised powder or as aqueous suspension into the sterile mixture obtained in step (v), and
(vii) adding the required amounts of water.

Usually, step (i) may be carried out by heating purified water to a temperature of 30-90°C, preferably 40-80°C, more preferably 50-70°C and adding the viscosity increasing agent under stirring. The dispersion is suitable for further processing when a homogeneous dispersion is obtained.

According to step (ii), the at least one isotonicity agent, the at least one chelator and the at least one preservative and optionally other carriers and/or diluents are dissolved in purified water having a temperature of 30-90°C, preferably 40-80°C, more preferably 50-75°C.

According to step (iii), the solution obtained in step (ii) is added to the dispersion obtained in step (i), preferably at product temperatures between 30-80°C, more preferably 40-70°C and even more preferably 45-70°C, under stirring.

In step (iv), the obtained mixture is adjusted to the desired pH, which is preferably in the range of 7.0-8.0, more preferably 7.3-7.7, and even more preferably 7.3-7.5. pH adjustment is preferably carried out using sodium hydroxide (NaOH) as 2 N aqueous solution.

According to step (v), the mixture obtained in step (iv) is sterilised, particularly sterilised under autoclave conditions at 120-128°C, preferably 121-128°C, preferably at 121°C for preferably 10-60 minutes, more preferably 20-40 minutes. Micronised sterile API, such as Brinzolamide, obtainable by the process described above is then added slowly into the mixture obtained in step (v) while stirring. The API, such as brinzolamide, is preferably used in form of micronised powder having a particle size distribution of 0.2 µm ≤ d_{90%} ≤ 5.0 µm, preferably 0.2 µm ≤ d_{90%} ≤ 4.0 µm and more preferably 1.0 µm ≤ d_{90%} ≤ 3.5 µm.

According to step (vii), the mixture obtained in step (vi) is diluted with water to the desired concentration. Subsequently, the obtained mixture can be filled into sterile primary containers suitable for the respective formulation.

The present invention is illustrated by the following examples.

### Examples

### Methods:

Particle size distribution (d_{90%}) has been determined by dynamic light scattering experiments using a Malvern Zetasizer.

The particle size of the brinzolamide micronised powder has been investigated by dynamic laser light scattering techniques after having suspended the powder formulation in an aqueous saturated brinzolamide solution using ultrasonic.

Mean particle size was determined via SEM: for each material sample the characterisation of the particles were based on the analysis of 150 particles selected randomly in five independent image fields from the sample of the material. The obtained particle sizes are averaged to give the mean particle diameter.

Quantitative analysis of degradation products has been performed via HPLC. Residue ethylene-oxide concentrations have been determined via gas chromatography.

### Sterilisation of brinzolamide by ethylene-oxide treatment

### Example 1:

Brinzolamide 15 g, manufactured according to WO 2010/103115 is packed in Tyvek pouch and placed into an appropriate ethylene oxide sterilization chamber (type one Europallet chamber) according to the manufacturer's instruction guide. Sterilization is conducted at a temperature of 45°C, at an relative humidity of 60%, and an ethylene oxide concentration of 565 mg/l. The contact time was about 180 minutes. After aeration in a well ventilated aeration chamber a sterile material having less than 0.15% degradation products is obtained.

### Example 2:

Brinzolamide, 1 kg, manufactured according to WO 2010/103115 is packed in double layer Tyvek pouches and placed into an appropriate ethylene oxide sterilization chamber (type one Europallet chamber) according to the manufacturer's instruction guide. Sterilization is conducted at a temperature of 55°C, at an relative humidity of 40%, and an ethylene oxide concentration of 380 mg/l. The contact time was about 75 minutes. After aeration in well ventilated aeration chamber a sterile material having less than 0.15% degradation products, less than 1 ppm of remaining ethylene oxide and less than 50 ppm of ethylene chlorohydrin is obtained.

### Example 3:

Brinzolamide, 1 kg, manufactured according to WO 2010/103115 is packed in double layer Tyvek pouches and placed into an appropriate ethylene oxide sterilization chamber (type one Europallet chamber) according to manufacturer's instruction guide. Sterilization is conducted at a temperature of 55°C, at an relative humidity of 40%, and an ethylene oxide concentration of 380 mg/l. The contact time was about 30 minutes. After aeration in well ventilated aeration chamber a sterile material having less than 0.15% degradation products, less than 1 ppm of remaining ethylene oxide and less than 50 ppm of ethylene chlorohydrin is obtained.

### Pharmaceutical compositions

The formulations A-C (see Table 1) are manufactured by the following procedure:
Purified water was heated in a stainless vessel to 65°C and added into the compounding vessel. The appropriate quantity of carbomer was added slowly to the hot purified water under stirring. Homogenization time and speed were adjusted in order to have homogeneous dispersions without agglomerates.

Purified water was heated to the temperature of approximately 70°C and added into a second compounding vessel. The following excipients of required amounts were added while stirring: mannitol, sodium chloride, disodium EDTA, benzalkonium chloride 50% w/v aqueous solution and polysorbate 80 or tyloxapol. Stirring was stopped to check for complete dissolution of the components.

The solution obtained was added to the carbomer mixture at product temperatures between 47-67°C. The reaction mixture was homogenised for further 10 minutes. Subsequently, the pH of the mixture was adjusted to 7.3-7.7 with sodium hydroxide 2 N aqueous solution.

The neutralized mixture was then sterilised at 121°C for 30 minutes using an autoclave. After cooling sterile brinzolamide micronised powder was added slowly while stirring. Finally, the necessary amount of purified water was added and the suspension was homogenised until obtaining an acceptable suspension which was checked by microscopic examination.

**Table 1: Pharmaceutical compositions**

| Ingredients | Formulations | | |
|---|---|---|---|
| | A (mg/ml) | B (mg/ml) | C (mg/ml) |
| sterile Brinzolamide | 10 | 10 | 10 |
| benzalkonium chloride | 0.10 | 0.10 | 0.10 |
| Polysorbate 80 | 0.25 | - | - |
| Tyloxapol | - | 0.25 | - |
| mannitol | 33.0 | 33.0 | 33.0 |
| Carbomer 974P | 4.0 | 4.0 | 4.0 |
| disodium EDTA | 0.10 | 0.10 | 0.10 |
| sodium chloride | 2.50 | 2.50 | 2.50 |
| sodium hydroxide /hydrochloric acid | qs to pH 7.5 | qs to pH 7.5 | qs to pH 7.5 |
| purified water | qs to 1 ml | qs to 1 ml | qs to 1 ml |

The mean diameter of the brinzolamide particles was about 1.0 ± 0.60 µm in case of formulation B and 1.46 ± 0.70 µm in case of formulation C. From the above it can be concluded that the presence of a surfactant (see formulation B) has almost no influence on the particle size.

Moreover, chemical and physical stability tests have been performed for pharmaceutical composition. No significant changes in the quality of brinzolamide in long term and accelerated test conditions for six months have been determined. Also, no significant differences in physical stability (measured by shelf life tests) and resuspendability tests could be observed in ongoing experiments.

From the above results, it becomes clear that the pharmaceutical formulations according to the present invention exhibit sufficient chemical and physical stability and suitable particle sizes for use in ophthalmic formulations. Surprisingly, even if formulating the pharmaceutical compositions without a surfactant, similar results compared to pharmaceutical compositions containing a surfactant are observed.

## Claims

1. A process for sterilizing an active pharmaceutical ingredient (API) comprising the steps of
(i) providing a API product, and
(ii) contacting said API product with ethylene oxide,
under conditions that at most 0.5 % by weight of degradation products are formed during step (ii).

2. The process of claim 1, wherein the API is brinzolamide, nepafenac or levocabastine hydrochloride, most preferably brinzolamide.

3. The process of any of claims 1-2, wherein step (ii) is carried out at temperatures in the range from 30-80°C, preferably 40-70°C, more preferably 50-60°C.

4. The process according to any of claims 1-3, wherein the concentration of ethylene oxide in step (ii) is in the range of 100-800 mg/l, preferably 200-600 mg/l and most preferably 300-450 mg/l, and wherein the contacting time in step (ii) is preferably in the range of 15-200 minutes, more preferably 20-100 minutes, most preferably 40-80 minutes.

5. A sterile API obtainable by a process according to any of claims 1-4.

6. The sterile API according to claim 5, which contains at most 10 ppm, preferably at most 3 ppm, more preferably at most 1 ppm of ethylene oxide.

7. The sterile API according to any of claims 5-6, which contains at most 0.5 % by weight, preferably 0.3 % by weight, more preferably at most 0.15 % by weight of degradation products.

8. A pharmaceutical composition, particularly for ophthalmic use, comprising the sterile API according to any of claims 5-7, and optionally at least one preservative, at least one isotonicity agent, at least one viscosity enhancing agent, at least one sequestering agent and water.

9. A pharmaceutical composition according to claim 8, wherein the preservative is selected from the group consisting of benzalkonium chloride, benzoic acid, benzyl alcohol, butylparaben, propylparaben, methylparaben, chlorobutanol, and phenoxyethanol, preferably benzalkonium chloride.

10. A pharmaceutical composition of any of claims 8-9, wherein the isotonicity agent is selected from the group consisting of dextrose, glycerol, mannitol, potassium chloride, and sodium chloride, preferably mannitol and sodium chloride.

11. A pharmaceutical composition according to any of claims 8-10, wherein the viscosity enhancing agent is selected from the group consisting of carbomer, poloxamer, polyvinyl alcohol, povidone, polyethylene oxide, carboxymethylcellulose calcium, carboxymethylcellulose sodium, hydroxyethylcellulose, hydroxypropylcellulose, hydroxymethylcellulose, hydroxypropylmethylcellulose, and methylcellulose, preferably carbomer.

12. A pharmaceutical composition according to any of claims 8-11, wherein the sequestering agent is a chelator, which forms a chelate complex with cations, preferably EDTA or salts thereof.

13. A pharmaceutical composition according to any of claims 8-12, which is free of surfactant.

14. A pharmaceutical composition according to any of claims 8-13, which is a suspension, particularly having a particle size distribution of 0.2 µm ≤ d_{90%} ≤ 5.0 µm, preferably 0.2 µm ≤ d_{90%} ≤ 4.0 µm, and more preferably 1.0 µm ≤ d_{90%} ≤ 3.5 µm.

15. The sterile API according to any of claims 5-7 or of the pharmaceutical composition according to any of claims 8-13 for the treatment of ocular hypertension and open-angle glaucoma.
